# EUROPEAN PATENT SPECIFICATION

(11) **EP 2 115 139 B1**
(45) Date of publication and mention of the grant of the patent: **28.01.2015**
(21) Application number: 08709296.1
(22) Date of filing: 29.01.2008
(51) Int. Cl.: C12N 15/13, C07K 16/28, C07K 16/40, C40B 50/00

(54) **METHOD FOR PRODUCING NOVEL IGE BASED REAGENTS**
VERFAHREN ZUR HERSTELLUNG NEUER REAGENTIEN AUF IGE-BASIS
PROCEDE DE FABRICATION DE NOUVEAUX REACTIFS A BASE D'IGE

(30) Priority: 29.01.2007 FI 20075059; 02.02.2007 US 887862 P; 05.10.2007 FI 20075707; 05.10.2007 US 977881 P
(43) Date of publication of application: 11.11.2009
(73) Proprietor: Teknologian tutkimuskeskus VTT, 02044 VTT (FI)
(72) Inventor: TAKKINEN, Kristiina, FI-02200 Espoo (FI); ROUVINEN, Juha, FI-80140 Joensuu (FI); NIEMI, Merja, FI-80130 Joensuu (FI); JYLHÄ, Sirpa, FI-02210 Espoo (FI); LAUKKANEN, Marja-Leena, FI-02210 Espoo (FI); SÖDERLUND, Hans, FI-02940 Espoo (FI)
(74) Representative: Matilainen, Mirja Helena
(86) International application number: PCT/FI2008/050027
(87) International publication number: WO 2008/092993

(56) References cited:
- EP-A1- 0 499 112
- WO-A1-97/04807
- WO-A1-99/62550
- WO-A2-2004/045512
- US-A- 5 965 709
- US-A- 6 063 621
- FLICKER SABINE ET AL: "Spatial clustering of the IgE epitopes on the major timothy grass pollen allergen Phl p 1: importance for allergenic activity.", THE JOURNAL OF ALLERGY AND CLINICAL IMMUNOLOGY JUN 2006 LNKD- PUBMED:16750995, vol. 117, no. 6, June 2006 (2006-06), pages 1336-1343, XP002620239, ISSN: 0091-6749
- JAKOBSEN CHARLOTTE G ET AL: "Isolation of high-affinity human IgE and IgG antibodies recognising Bet v 1 and Humicola lanuginosa lipase from combinatorial phage libraries.", MOLECULAR IMMUNOLOGY AUG 2004 LNKD- PUBMED:15302157, vol. 41, no. 10, August 2004 (2004-08), pages 941-953, XP002620240, ISSN: 0161-5890
- LAUKKANEN MARJA-LEENA ET AL: "Hevein-specific recombinant IgE antibodies from human single-chain antibody phage display libraries.", JOURNAL OF IMMUNOLOGICAL METHODS JUL 2003 LNKD- PUBMED:12957414, vol. 278, no. 1-2, July 2003 (2003-07), pages 271-281, XP002620241, ISSN: 0022-1759
- DE GENST ERWIN ET AL: "Molecular basis for the preferential cleft recognition by dromedary heavy-chain antibodies.", PROCEEDINGS OF THE NATIONAL ACADEMY OF SCIENCES OF THE UNITED STATES OF AMERICA 21 MAR 2006 LNKD- PUBMED:16537393, vol. 103, no. 12, 21 March 2006 (2006-03-21), pages 4586-4591, XP002620494, ISSN: 0027-8424
- NIEMI MERJA ET AL: "Molecular interactions between a recombinant IgE antibody and the beta-lactoglobulin allergen.", STRUCTURE (LONDON, ENGLAND : 1993) NOV 2007 LNKD- PUBMED:17997967, vol. 15, no. 11, November 2007 (2007-11), pages 1413-1421, XP002620242, ISSN: 0969-2126
- SCHEFFER G.L. ET AL.: 'Specific detection of multidrug resistance MRP1, MRP2, MRP3, MRP5, and MDR3 P-glycoprotein with a panel of monoclonal antibodies' CANCER RESEARCH vol. 60, 15 September 2000, pages 5269 - 5277, XP002249637
- BINYAMIN L. ET AL.: 'Targeting an extracellular epitope of the human multidrug resistance protein 1 (MRP1) in malignant cells with a novel recombinant single chain Fv antibody' INT. J. CANCER vol. 110, 2004, pages 882 - 890, XP003023005
- STEINBERGER P. ET AL.: 'Construction of a combinatorial IgE library from an allergic patient' THE JOURNAL OF BIOLOGICAL CHEMISTRY vol. 271, no. 18, 03 May 1996, pages 10967 - 10972, XP002950279
- HOOGENBOOM H.R.: 'Selecting and screening recombinant antibody libraries' NATURE BIOTECHNOLOGY vol. 23, no. 9, September 2005, pages 1105 - 1116, XP002348401
- GERK P.M. ET AL.: 'Estradiol 3-glucuronide is transported by the multidrug resistance-associated protein 2 but does not activate the allosteric site bound by estradiol 17-glucuronide' DRUG METABOLISM AND DISPOSITION vol. 32, no. 10, 2004, pages 1139 - 1145, XP003023006
- BORST P. ET AL.: 'MRP2 and 3 in health and disease' CANCER LETTERS vol. 234, 2006, pages 51 - 61, XP005324059

### SEQUENCE LISTING

<110> Valtion teknillinen tutkimuskeskus
<120> Method for producing novel IgE based reagents
<130>
<160> 3
<170> PatentIn version 3.1
<210> 1
   <211> 8
   <212> PRT
   <213> Artificial
<220>
   <221> MISC_FEATURE
   <222> (5) .. (5)
   <223> X is any amino acid
<220>
   <221> MISC_FEATURE
   <222> (8) .. (8)
   <223> X is Ser or Thr
<400> 1
   Leu Leu Ile Tyr Xaa Ala Ser Xaa
   1               5
<210> 2
   <211> 11
   <212> PRT
   <213> Artificial
<220>
   <221> MISC_FEATURE
   <222> (3) .. (5)
   <223> X is any amino acid
<220>
   <221> MISC_FEATURE
   <222> (8) .. (8)
   <223> X is Ser or Thr
<220>
   <221> MISC_FEATURE
   <222> (9) .. (11)
   <223> X is any amino acid
<400> 2
   Leu Leu Xaa Xaa Xaa Ala Ser Xaa Xaa Xaa Xaa
   1               5                   10
<210> 3
   <211> 11
   <212> PRT
   <213> Artificial
<400> 3
   Leu Leu Ile Tyr Ala Ala Ser Ser Leu Gln Ser
   1               5                   10

## Description

### Field of the invention

This invention relates to protein engineering technology. More particularly, the present invention relates to a method for preparing human IgE antibodies and derivatives thereof, which bind epitope structures that are weakly IgG or IgM immunoreactive.

### Background of the Invention

Antibodies are today the most potent and rapidly growing drug class for human therapy. FDA has approved 18 antibodies for therapeutic use in the United States for treatment of different diseases, such as cancer, inflammation, transplantation and infections (Carter 2006). New antibody generation techniques based on the use of antibody phage display libraries or transgenic mice allowing isolation of fully human antibodies ideal for therapeutic applications have speed up the development process substantially. The current market size of therapeutic antibodies ~ $15 billion is estimated to exceed to $30 billion by 2010. Rapid progress in genomics, transcriptomics, proteomics and interactomics is revolutionizing the identification of therapeutic targets requiring development of antibodies recognizing specifically these targets with desired action mechanism, e.g., inhibition or activation of protein/ligand or protein/protein interactions.

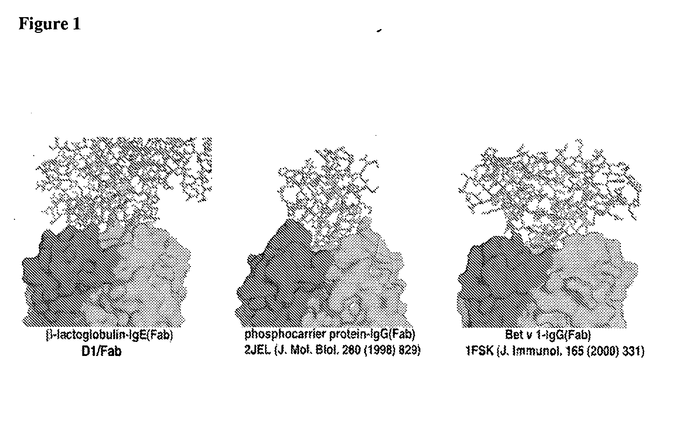

An epitope is a localized region on the surface of an antigen to which an antibody binds, epitopes can be composed of sugars, lipids or amino acids. Most epitopes recognized by antibodies are three-dimensional surface features of an antigen molecule. Exceptions are linear epitopes, which are determined by the amino acid sequence (the primary structure) rather than by the 3D, tertiary, structure of a protein. The 82 different antigen-antibody immuncomplexes available at present in the Protein Data Bank represent almost exclusively IgG-antigen complexes. The analysis of the structural elements in the IgG epitopes (alpha-helices, beta-strands, and loops) and the shapes of epitopes (convex, planar, concave) shows that half of the epitopes are formed by loops alone and another half contains both loops and secondary structure elements (see Fig. 1). The majority of the IgG epitopes (68 %) are located in the convex or exposed loop regions of antigens. The clefts and/or depressions on protein structures and planar surfaces are thus not preferential IgG epitope stuctures. Recently identified antibody classes from dromedaries and sharks, so called single domain antibodies having only the VH region with protruding HCDR3 loops, are able to recognize and bind cleft regions, such as enzyme substrate sites (De Genst *et al.* 2006). However, these antibodies are not human origin and thus are not preferential for therapeutic applications. Human antibodies with the capability to recognize weakly immunogenic structures, clefts and planar surfaces, would offer a valuable source to develop novel binders for human therapy and diagnostics.

DE GENST ERWIN ET AL: "Molecular basis for the preferential cleft recognition by dromedary heavy-chain antibodies.", PROCEEDINGS OF THE NATIONAL ACADEMY OF SCIENCES OF THE UNITED STATES OF AMERICA, 21 MAR 2006, vol. 103, no. 12, 21 March 2006 (2006-03-21), pages 4586-4591, discloses a method for preparing dromedary heavy-chain antibodies for cleft recognition.

The recently solved structure of an IgE Fab antibody in complex with an allergen, β-lactoglobulin (BLG, Bos d 5) structure suggests that IgE antibodies prefer structurally different binding sites than IgG antibodies (in press). This discovery opens possibilities for novel concepts in immunotherapy and diagnostics. The CDR loops of the IgE/Fab light chain are responsible for the binding of a flat beta-sheet region of BLG. This IgE-epitope is strikingly different when compared to IgG-epitopes that are located normally in the exposed loop regions of antigens. Furthermore, the IgE VH region and especially the HCDR3 loop is structurally different when compared to IgG antibodies: it is forming a loop structure that is recognising a cavity on the allergen surface. Based on this observation it should be possible to develop chimeric human antibodies, consisting of IgE V-regions grafted onto the constant part of an antibody of the IgG or IgM type, for those therapeutic targets where the binding specificity is required towards a planar surface or a flat beta-sheet and structures that are not exposed on the protein surface (e.g., substrate binding sites of enzymes and drug resistance pumps).

### Summary of the Invention

The present invention relates to a method for preparing human IgE antibodies and derivatives thereof, which bind to structures that are not exposed on the protein surface, such as depressions, clefts or channels or alternatively to flat surfaces mediating protein-protein interactions of resistance transporters.

This invention thus provides a method to produce new reagents to be utilised in different kinds of immunoassay protocols, as well as in human immunotherapy and construction of focused antibody libraries. The invention also permits guaranteed continuous supply of these specific reagents of uniform quality, eliminating inherent batch-to-batch variation of polyclonal antisera. These advantageous effects permit the manufacture of new, specific and economical immunoreagnets of uniform quality.

Consequently, one specific object of the present method is to provide human IgE monoclonal antibodies, fragments thereof, or other derivatives of such antibodies, which bind target proteins with affinity and specificity high enough to allow their qualitative and quantitative measurement and imaging in biological samples, as well as their use in immunotherapy. The antibodies obtained by the present method demonstrate a specific binding to therapeutic or diagnostic targets in a desired mode, which is not within reach of monoclonal antibodies developed from other sources.

A further object of this invention is to provide methods of using structural data obtained for constructing focused IgE antibody libraries towards therapeutic and diagnostic targets where the binding specificity is towards areas of protein structures that are weakly IgG / IgM immunoreactive.

Other objects, features and advantages of the present invention will become apparent from the following drawings and detailed description. It should be understood, however, that the detailed description and the specific examples, while indicating preferred embodiments of the invention, are given for illustration only, since various changes and modifications within the spirit and scope of the invention will become apparent to those skilled in the art from this detailed description.

### Brief Description of the Drawings

**Figure 1****.** Comparison of the epitope strucutures of the IgE D1 Fab-allergen and IgG-antigen immunocomplexes. The binding of the D1 IgE Fab to β-lactoglobulin (left), the IgG antibody-antigen type binding IgG Fab JEL42 to phosphocarrier protein (middle) (Prasad *et al.* 1998) and IgG-allergen type binding of the BV16/Fab to the pollen allergen Bet v 1. (right) (Mirza *et al*, 2000).

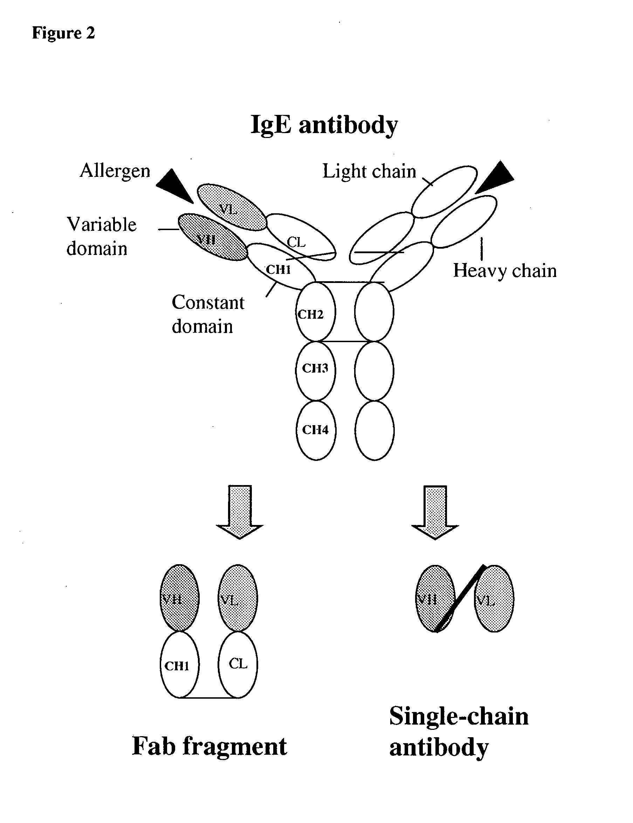

**Figure 2** shows a schematic presentation of an intact human IgE subclass antibody, Fab fragment and single-chain antibody (scFv). The antigen-binding site is indicated by a triangle.
**Figure 3** shows schematically the panning procedure.

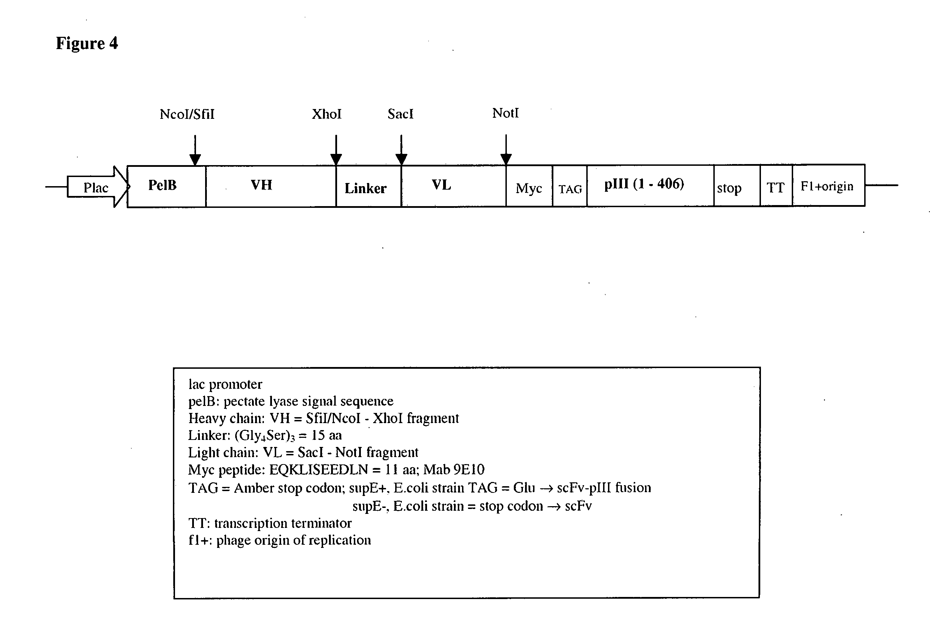

**Figure 4** shows a schematic presentation of the scFv phage display vector used for the construction of scFv phage libraries.

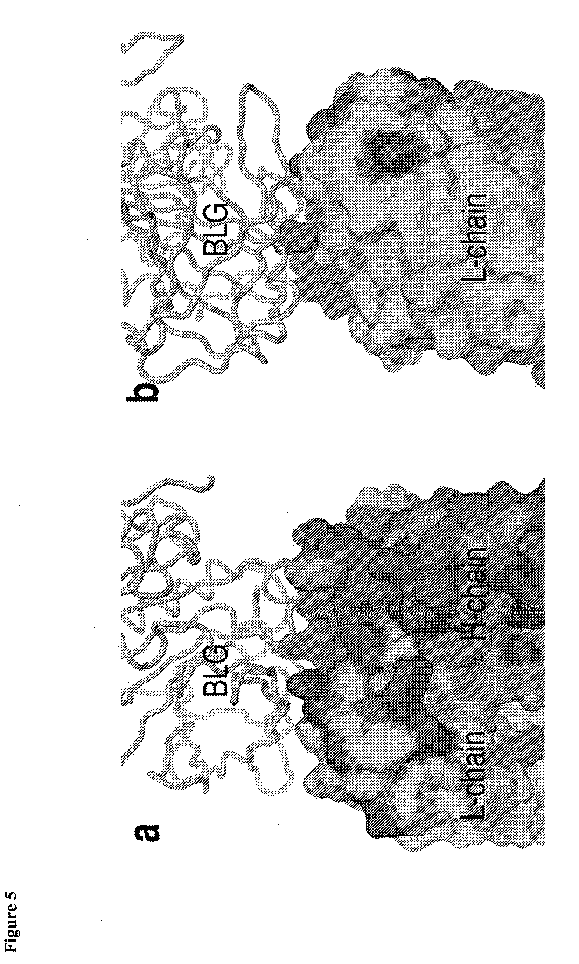

**Figure 5** shows the surface of D1/Fab antibody and ribbon model of allergen BLG. In this figure identical residues of the D1/Fab with hevein-binding IgE-antibody (clone IC2) (Laukkanen *et al.* 2003) are shown in light grey, different residues are in dark grey; a) front view, b) side view

The figures of the constructions are not in scale.

### Abbreviations

- cDNA: complementary deoxyribonucleic acid
- CDR: complementarity determining region
- DNA: deoxyribonucleic acid
- *E. colt*: *Escherichia coli*
- EDTA: ethylenediamine tetraacetic acid
- ELISA: enzyme-linked immunosorbent assay
- Fab: fragment with specific antigen binding
- Fd: variable and first constant domain of a heavy chain
- Fv: variable regions of an antibody with specific antigen binding
- IgE: immunoglobulin E
- mRNA: messenger ribonucleic acid
- MRP2: multidrug resistance associated protein 2
- MRP1: multidrug resistance associated protein 1
- PCR: polymerase chain reaction
- RNA: ribonucleic acid
- scFv: single-chain antibody
- TEA: triethanolamine
- V_{H}: variable region of a heavy chain
- V_{L}: variable region of a light chain

### Detailed Description of the Invention

The following definitions are provided for some terms used in this specification. The terms, "immunoglobulin", "heavy chain", "light chain" and "Fab" are used in the same way as in the European Patent Application No. 0125023.

"Antibody" in its various grammatical forms is used herein as a collective noun that refers to a population of immunoglobulin molecules and/or immunologically active portions of immunoglobulin molecules, i.e., molecules that contain an antigen binding site or a paratope.

An "antigen-binding site", a "paratope", is the structural portion of an antibody molecule that specifically binds an antigen.

Exemplary antibodies are those portions of an immunoglobulin molecule that contain the paratope, including those portions known as Fab and Fv.

"Fab" (fragment with specific antigen binding), a portion of antibodies can be prepared by the proteolytic reaction of papain on substantially intact antibodies by methods that are well known. See for example, U.S. Patent No. 4,342,566. Fab fragments can also be produced by recombinant methods, which are well known to those skilled in the art. See, for example, U.S. Patent 4,949,778.

"Domain" is used to describe an independently folding part of a protein. General structural definitions for domain borders in natural proteins are given in Argos, 1988.

A "variable domain" or "Fv" is used to describe those regions of the immunoglobulin molecule, which are responsible for antigen or hapten binding. Usually these consist of approximately the first 100 amino acids of the N-termini of the light and the heavy chain of the immunoglobulin molecule.

"Single-chain antibody" (scFv) is used to define a molecule in which the variable domains of the heavy and light chain of an antibody are joined together via a linker peptide to form a continuous amino acid chain synthesised from a single mRNA molecule (transcript).

"Linker" is used to describe an amino acid sequence that extends between adjacent domains in a natural or engineered protein.

The term "IgE producing cells" refers, e.g., to white blood cells such as lymphocytes.

The term"human derived sample" refers preferably to a blood or serum sample taken from human patient, preferably patient suffering from an allergy.

The term "receptor protein" refers to a protein which is able to bind a ligand and this ligand/receptor relationship is related to biologically relevant activity of a cell. An example of such ligand/receptor relationship is acetylcholine (a ligand) and acetylcholine receptors, such as nicotinic and muscarinic cholinergic receptors, which respond to the binding of acetylcholine by changing their biological activity. Said ligand may also be another protein.

### Human antibody (scFv, Fab or whole antibody) libraries containing the human IgE VH-regions

The IgE VH-region of the D1 IgE Fab and especially the HCDR3 loop are structurally different when compared to IgG antibodies (see Fig. 1). Based on this observation it should be possible to develop human IgE VH-region containing antibodies for those therapeutic targets where the binding specificity is required towards protein structures that are not exposed on the surface, e.g., that are weakly IgG/IgM immunoreactive, such as receptors, drug resistance pumps and substrate binding sites of enzymes (De Genst *et al*. 2006). A diverse IgE VH-pool from human lymphocytes is used as a building block to construct a functional human antibody library in a scFv, Fab or whole antibody format. Resulting libraries are selected against therapeutic targets requiring specific recognition of structures that are not immunoreactive against IgG/IgM, e.g. cleft structures or planar surfaces. Therefore, the object of the present invention is to provide a method of preparing a recombinant IgE monoclonal antibody or a functional fragment thereof capable of preventing binding of a ligand or substrate to a receptor protein or enzyme, said method comprising the steps of
a) isolating total mRNA from IgE producing cells from a human derived sample;
b) synthesizing of the cDNAs encoding the IgE Fd gene region and kappa/lambda light chain genes based on the total mRNA obtained in step a) to create an IgE expression library;
c) screening the expressed library against a desired target protein or cell or particle expressing said protein on its surface, wherein said protein is a receptor protein or an enzyme;
d) isolating clones from the library showing medium or high affinity (i.e. over 10⁷M⁻¹) towards said protein;
e) selecting those clones obtained from step d) that prevent the binding of a ligand to a receptor protein or the binding of a substrate to an enzyme;
f) optionally isolating the DNA encoding the IgE antibody obtained in step e).

Preferably, said method further comprises a step of g) converting a clone obtained from step e) into an antibody of the IgG or IgM type. It is well-known in the art that the constant parts of an antibody can be changed to alter the type of the antibody.

One preferred embodiment of the present invention is that the target protein of interest in the present method is a drug-resistance pump, such as multidrug resistance associated protein 2, MRP2. Said drug-resistance pumps are major canalicular organic anion transporters moving anions across the cell membrane. Said pumps may have several different ligands. The present invention is preferably directed to method for preparing IgE based compounds that prevent binding of one or more of these different ligands but not all to said pump having several ligands. More preferably, said IgE based compound prevents binding of one specific ligand to said pump having several ligands so that the other ligands may bind the pump while the binding of said specific ligand is prevented by the binding of IgE based compound to said pump. Thus, the method of the invention preferably comprises a further step of selecting an IgE monoclonal antibody, which binds to a drug-resistance pump having several ligands so that the binding of all of said several ligands is not prevented or blocked. More preferably, said step comprises selecting an IgE monoclonal antibody, which binds to a drug-resistance pump having several ligands so that the binding of one specific ligand is prevented or blocked while the other ligands can bind to the pump while said IgE monoclonal antibody is bound to said pump. The same method can be performed with an enzyme having several substrates.

Another preferred embodiment of the present invention is selecting an IgE monoclonal antibody, which binds to a drug-resistance pump having several ligands so that the binding of all ligands is prevented or blocked.

Another preferred embodiment of the present invention is selecting an IgE monoclonal antibody, which binds to a first receptor protein having one or several ligands but do not bind to a second receptor protein having one or several ligands, which second receptor protein is related to the first receptor protein and may also have one or more ligands. For instance, if said target protein is multidrug resistance associated protein 2, MRP2, which is a drug-resistance pump having several ligands, then it is preferable to select and isolate an IgE monoclonal antibody, which prevents the binding of one or more ligands to MRP2, but do not prevent binding of a ligand or ligands to a related receptor protein, such as ATP-binding cassette transporter (i.e. ABC-transporter). In this context, the term "related receptor protein" refers to a group of receptor proteins, which have structural homology, overlapping ligand or substrate specificity and/or a similar type of function in a cell or tissue. Other related receptor protein to MRP2 is, e.g., multidrug resistance associated protein 1, MRP1. The substrate/ligand specificities of MRP1 and MRP2 overlap to a large extent but their tissue localisation differ. Known ligands for MRP2 is conjugated or unconjugated organic anion, such as glutathione conjugates, glucuronide conjugates (e.g., estradiol-17-b-D-glucuronide), leukotrines, methotrexate, ochratoxin A and p-aminohippurate, PAH.

As an example of one preferred embodiment of the invention, IgE monoclonal antibody capable of preventing the binding of one or more ligands to multidrug resistance associated protein 2, MRP2, can be prepared in the following way: The human IgEVH/κVL or λVL scFv-phage library is first constructed from mRNAs isolated from lymphocytes of allergic patients. The variable region of the light and heavy chain cDNAs are synthesised using human IgE-specific primers for Fd cDNAs and human kappa (κ) and lambda (λ) light chains using human κ and λ chain specific primers. The variable regions of the light and heavy chains are amplified by PCR using human κ and λ chain specific primers for Vκ and Vλ cDNAs and human IgE specific primers for V_{H} cDNAs, respectively. The human IgE/IgG scFv library are then constructed by cloning the variable region cDNAs into a scFv phage display vector using restriction sites introduced into the PCR primers.

The human IgEVH/κVL or λ VLscFv library is then selected by phage display using a biopanning procedure against MRP2 containing vesicles (for a review of MRP2, see Borst et al. 2006). MRP2 vesicles are preferably passively immobilised on to microtitre well. The elution of phages is preferably done with TEA. The phage eluate is amplified in *E. coli* cells. After sufficient rounds of biopanning, the binding specificity of the selected scFv fragments is analysed by ELISA. Several MRP2 -specific scFv fragment clones are likely obtained.

As described herein, the phage display technique is an efficient and feasible approach to develop human IgEVH/κVL or λVL recombinant anti-MRP2 antibodies for the selection steps of the present invention. Isolated anti-MRP2 antibodies have high affinity and specificity towards MRP2 providing their use to characterize MRP2 function by ligand-activity measurements and distribution by immunochemical staining methods from cell and tissue samples. Furthermore, isolated anti-MRP2 antibodies may provide tools for fine-tuning of MRP2 function, such as inactivation or activation of efflux of different ligands of the transporter. Isolated IgE antibodies can be further applied to selective inactivation / activation assays with target receptors, transporters or enzymes and specific ligands thereof in order to find a clone having no effect to closely related counterparts on cells, tissues or organs.

### Focused IgE-antibody library towards flat structures

One of the most challenging problem in drug discovery is the "undruggable" targets, i.e. biological macromolecules formed by protein-protein interactions. Many types of protein-protein complexes including homo- and heterodimers, enzyme substrate or inhibitor complexes, antibody-antigen complexes or multicomponents, e.g., ribosome or proteosome, mediate important functions in cells, organelles, tissues and organs. These protein-protein interactions generally involving large and relatively flat surface areas with numerous contact sites are difficult targets for small molecule drugs.

The recently solved structure of an IgE Fab antibody (D1 Fab) in complex with an allergen, β-lactoglobulin (BLG, Bos d 5) structure suggests that IgE antibodies prefer structurally different binding sites than IgG antibodies (in press). The epitope of BLG consists of six different short fragments of the polypeptide chain, which are located almost exclusively in the secondary structure elements, especially in the β-strands, covering a flat area on the allergen surface. All six CDR (complementary determining region) loops of the IgE/Fab fragment participates in the binding of BLG. The CDR loops of the IgE/Fab light chain are responsible for the binding of the flat β-sheet region of BLG. The amino acid sequence comparison of published IgE sequences reveals that the light chains of the known IgE antibodies binding to diverse groups of allergens are strikingly conserved (see Table V), suggesting that these similar light chain sequences could also bind a flat surface of a β-sheet or a similar flat patch. This gives tools to construct focused libraries that can be utilised for the isolation of antibodies specific to flat surfaces applicable in the diagnostics or therapy. The conserved light chain sequence information is used to construct a limited pool of light chains or a single light chain with the characteristic amino acid sequences identified in the IgE antibodies. This light chain sequence information is combined with a diverse pool of IgE heavy chain genes isolated from lymphocytes of several allergic patients. The resulting antibody phage display library, in either scFv or Fab display format, is used to select target specific IgE antibodies essentially as described in Example I or as described in Hoogenboom *et al*. (1998) and Hoogenboom (2005).

While one successful selection strategy for obtaining antibody fragments of the invention has been described, numerous variations, by which antibody fragments of the invention may be obtained, will be apparent to those skilled in the art. It may prove possible to select scFv fragments of the invention directly from a phage or microbial display library of scFv fragment or its derivatives. A phage or microbial cell, which presents a scFv fragment or other antibody fragment of the invention as a fusion protein with a surface protein, represents a still further aspect of the invention. It is also possible to isolate the MRP-specific antibodies using flow cytometry.

While microbial expression of antibodies and antibody derivatives of the invention offers means for efficient and economical production of highly specific reagents of uniform quality suitable for use in immunodiagnostic assays and immunotherapy, alternatively it may prove possible to produce such a reagent, or at least a portion thereof, synthetically. By applying conventional genetic engineering techniques, initially obtained antibody fragments of the invention may be altered, e.g. new sequences linked, without substantially altering the binding characteristics. Such techniques may be employed to produce novel binding hybrid proteins, which retain both affinity and specificity for the target antigen as defined hereinbefore.

In another aspect, the present invention also provides DNA molecules encoding an antibody or antibody derivative of the invention, and fragments of such DNAs, which encode the CDRs of the V_{L} and/or V_{H} region. Such a DNA may be cloned in a vector, more particularly, for example, an expression vector which is capable of directing expression of antibody derivatives of the invention, or at least one antibody chain or a part of one antibody chain.

In a further aspect of the invention, host cells are provided, selected from bacterial cells, yeast cells, fungal cells, insect cells, plant cells and mammalian cells, containing a DNA molecule of the invention, including host cells capable of expressing an antibody or antibody derivative of the invention. Thus, antibody derivatives of the invention may be prepared by culturing host cells of the invention expressing the required antibody chain(s), and either directly recovering the desired protein or, if necessary, initially recovering and combining individual chains.

### EXAMPLE 1

### I. Construction of the human IgE/IgM scFv phage libraries

Previously constructed human naive scFv libraries (IgM/kappa and IgM/lambda) and milk and latex allergic IgE scFv libraries (IgE/kappa and IgE/lambda) were used as a starting material for the construction of IgE/IgM libraries. Briefly, the human naive libraries (IgM) were constructed from the lymphocyte isolated from 50 healthy blood donors. For the construction of the IgE libraries altogether 150 ml of heparinised blood was obtained from three allergic patient with different allergenic profiles. Lymphocytes were isolated according to an Ig-Prime kit protocol (Novagen). Per 10 ml of blood 30 ml of lysis buffer (155 mM NH₄Cl, 10 mM NH₄HCO₃, 0.1 mM EDTA, pH 7.4) was added and incubated on ice for 15 min with shaking occasionally. After centrifugation at 450 g for 10 min the lymphocytes, i.e. the white blood cell pellet, were collected. The pellet was washed twice with lysis buffer and after the final centrifugation the lymphocyte pellet was resuspended in D-solution. Lymphocyte RNAs were isolated using Promega's RNAgents Total RNA Isolation kit according to the manufacturer's protocol. The first strand cDNA synthesis was carried out using Promega's Reverse Transcription system kit. For the synthesis of Fd-fragment cDNA and light chain cDNAs the primers of the constant region of the epsilon (ε) chain (Cε1) and the primer of the kappa (Cκ1) and lambda (Cλ1) chain were used, respectively. Primers used for the cDNA synthesis and PCR amplifications of human IgE Fd region and light chains are showed in Table I and Table II.

PCR amplifications were carried out in two steps: a primary PCR for amplifying Fd and light chains from cDNA templates and a secondary PCR for adding restriction sites to the 5'-end of the DNA fragments obtained after a primary PCR. First the Fd region was amplified by PCR using the primers specific for the variable region of the heavy chains (VH1a-VH7a) and Cε1 primer. Accordingly, the kappa and lambda light chains were amplified using specific primers for variable region of the light chains (Vκ1a-Vκ6b and Vλ1a-Vλ10) and Cκ/λ1 primer, respectively. Primers for the secondary PCR were Cκ1 and Vκ/λ1 and Cκ for the kappa light region, Vκ/λ1 and Cλ1 for the kappa light chain and Vλ1A and Cκ/λ1 for the lambda light chain. The primary PCR amplification was done at the following conditions: 1 cycle of 3 min at 93°C for denaturation, 7 cycles of 1 min at 93°C, 30 s at 63°C and 50 s at 58°C for annealing and 1 min at 72°C for elongation, 23 cycles of 1 min at 93°C, 30 s at 63°C and 1 min at 72°C followed by 1 cycle of 10 min at 72°C. For the secondary PCR the amplification conditions were as follows: 1 cycle of 3 min at 95°C for denaturation, 25 cycles of 1.5 min at 94°C, 1 min at 65°C for annealing and 1.5 min at 72°C for elongation followed by 1 cycle of 10 min at 72°C. Between the primary and the secondary PCR and after the secondary PCR the amplified DNA fragments were purified.

The final PCR products of the different antibody fragments were pooled and digested with appropriate restriction enzymes. Digested DNA fragments, encoding IgE Fd region and κ and λ light chains, were ligated into a phagemid vector and transformed into *E*. *coli* XL-1 Blue cells to yield an Fab-κ and Fab-λ libraries of 10⁶ independent clones. To avoid possible problems on the expression of Fab fragments on a phage particle an antibody library in scFv format was constructed. Phagemid DNAs from different libraries were isolated and used as template DNAs for amplifying the variable regions of the human IgE heavy and human light chains in order to construct human IgE scFv-κ and scFv-λ libraries.

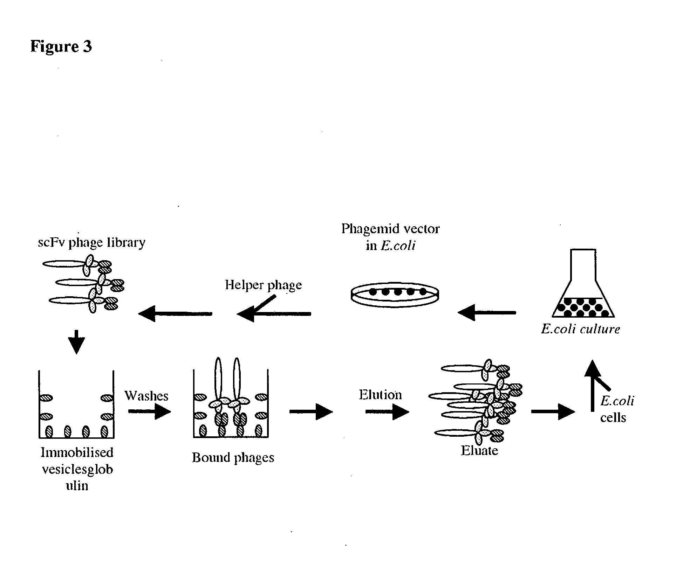

PCR amplification of the variable region of the heavy chain was carried out using human V_{H} specific primers (VH1-VH4 and VH1A). Amplification of the variable region of the light chains was done using the following primer pairs: Vκ1-Vκ7, Vκ2-Vκ8, Vκ3-Vκ9, Vκ4-Vκ10, Vκ5-Vκ11 and Vκ6-Vκ11 for human kappa chain and Vλ1-Vλ8, Vλ2-Vλ9, Vλ3-Vλ9, Vλ4-Vλ9, Vλ5-Vλ10, Vλ6-Vλ10 and Vλ7-Vλ10 for human lambda chain (see Tables III and IV). The amplified DNA fragments were purified and digested in order to ligate into a scFv phage display vector (Fig.3). Ligation mixtures were transformed into *E*. *coli* XL-1 Blue cells resulting in the human IgE scFv-κ and scFv-λ libraries with approximately 10⁵ independent clones.

Finally the cDNAs encoding the variable regions of kappa and lambda chain of the naive libraries were digested with *Sac*I and *Not*I restriction enzymes. Then the DNA fragments were ligated into the *Sac*I*-Not*I digested vectors containing the cDNAs encoding the IgE variable regions from milk and latex allergic patient separately (IgE/IgM milk and IgE/IgM latex). Then the resulting plasmids were transformed into *E.coli* XL-1 Blue cells. For the isolation of library plasmid DNAs the cells containing the IgE/IgM milk and IgE/IgM latex plasmid DNAs were pooled together in order to result in the human IgE/IgM kappa and IgE/IgM lambda libraries.

### II Vesicle preparation

The insect cells infected with the baculovirus containing the cDNA encoding the MRP2 transporter were harvested (1000 g, +4°C, 10min). The cell pellet was washed with cold PBS and re-centrifugated. The cell pellet was washed twice with harvest buffer (50 mM Tris-HCl, pH 6.8, 300 mM mannitol, protease inhibitor cocktail) followed by the centrifucation (800 g, +4°C, 5min). Then the cell pellet was resuspended in membrane buffer (50 mM Tris-HCl, pH 6.8, 50 mM mannitol, 2 mM EDTA, pH 8.0, protease inhibitor cocktail) and homogenised followed by the 1-hour incubation on ice and centrifucation (800 g, +4°C, 10min). The supernatant was subjected to the ultracentrifucation (100 000 g, +4°C, 60min). The vesicle pellet was resuspended in to the membrane buffer and homogenised using a syringe and a G27 needle.

### III. Selection of human IgE/IgM libraries on MRP2 displaying vesicles

The vesicles with and without the MRP2 in Buffer A (50 mM MOPS-TrisHCl, pH 7.0, 70 mM KCl, 7.5 mM MgCl₂) were immobilised onto microtitre plate wells. After blocking of the wells with Buffer A -1%BSA the depletion of the phage pool was carried out. The phages of the IgE/IgM kappa and lambda libraries were combined and diluted 1:4 into Buffer B (50 mM MOPS-TrisHCl, pH 7.0, 70 mM KCl, 7.5 mM MgCl₂, 1% BSA) and β-estradiol and MgATP were added to a final concentration of 50 µM and 4 mM, respectively. Then the phages binding to the vesicles without MRP2 were depleted by the incubation of the phage pool with the vesicles without the MRP2 for 3 h at +37°C. The unbound phages were recovered (depleted phage pool).

In the selection the depleted phage pool was incubated with both vesicles. The enrichment of the specific binders was followed by the incubation of the depleted phage pool with vesicles without MRP2. First, the depleted phage pool was diluted 1:2 in Buffer B and β-estradiol and MgATP were added as above and then subjected for the incubation with vesicles. The bound phages were eluted with TEA. Finally the *E.coli* XL-1 Blue cells were infected with the eluted phages for the amplification of the enriched phages.

### IV. Characterisation of the isolated antibodies

The characterisation of the antibody binding properties to MRP2 were carried out by ELISA. The enriched antibody phage pools and/or antibody phages prepared from the individual clones. In ELISA, the vesicles with and without the MRP2 in Buffer A (50 mM MOPS-TrisHCl, pH 7.0, 70 mM KCl, 7.5 mM MgCl₂) were immobilised onto microtitre plate wells. After blocking of the wells with Buffer A -1%BSA the phages were added and incubated for 1 h at RT with shaking. After the washing step the anti-M13 antibody was used for the detection of the bound phages. As a secondary antibody AFOS-conjugated anti-mouse IgG (H+L) were used. After addition of the substrate, *p-*nitrophenylphosphatase, the absorbance values were read at 405 nm.

The transportation of the substrates such as β-estradiol across the cell membrane was carried out using vesicular transport assay that determinates the interaction of test drugs (activators/inhibitors) with the MRP2 transporter and/or related ATP-binding cassette transporters. The selected scFv antibody clones (different concentrations) were incubated with the MRP2 vesicles and control vesicles prepared as described above. Both vesicles were diluted into the assay buffer (40 mM MOPS-TrisHCl, pH 7.0, 55 mM KCl, 6 mM MgCl₂). Then the labelled substrate e.g. ³H β-estradiol 17-(β-D-glucuronide) for the final concentration of 50 mM was added and pre-incubated for 5 min at +37°C. The reaction was started by the addition of Mg-ATP for the final concentration of 4 mM. The reaction was let to proceed for 16 min at +37°C. The vesicles were collected on glass fiber filters (pore size 0.7 µm) and washed before measurement on liquid scintillation system.

**TABLE I: Primers used for cDNA synthesis and PCR amplification of the human IgE Fd region.**

| |
|---|
| Cε1: 5'- GCTGAAGGTTTTGTTGTCGACCCAGTC -3' |
| CεNotI: 5'- GAATGGTGCGGCCGCGCTGAAGGTTTTGTTGTCG -3' |
| VH1a: 5'- ATGGCCGCAGCTCAGGTKCAGCTGGTGCAG -3' |
| VH1b: 5'- ATGGCCGCAGCTCAGGTCCAGCTTGTGCAG -3' |
| VH1c: 5'- ATGGCCGCAGCTSAGGTCCAGCTGGTACAG -3' |
| VH1d: 5'- ATGGCCGCAGCTCARATGCAGCTGGTGCAG-3' |
| VH2a: 5'- ATGGCCGCAGCTCAGATCACCTTGAAGGAG -3' |
| VH2b: 5'- ATGGCCGCAGCTCAGGTCACCTTGARGGAG -3' |
| VH3a: 5'- ATGGCCGCAGCTGARGTGCAGCTGGTGGAG -3' |
| VH3b: 5'- ATGGCCGCAGCTCAGGTGCAGCTGGTGGAG -3' |
| VH3c: 5'- ATGGCCGCAGCTGAGGTGCAGCTGTTGGAG -3' |
| VH4a: 5'- ATGGCCGCAGCTCAGSTGCAGCTGCAGGAG -3' |
| VH4b: 5'- ATGGCCGCAGCTCAGGTGCAGCTACAGCAG -3' |
| VH5a: 5'- ATGGCCGCAGCTGARGTGCAGCTGGTGCAG -3' |
| VH6a: 5'- ATGGCCGCAGCTCAGGTACAGCTGCAGCAG -3' |
| VH7a: 5'- ATGGCCGCAGCTCAGGTSCAGCTGGTGCAA -3' |
| VH1A: 5'- TTACTCGCGGCCCAGCCGGCCATGGCCGCAGCT -3' |

**TABLE II: Primers used for cDNA synthesis and PCR amplification of human kappa and lambda chains.**

| |
|---|
| Cκ1: 5'- AGGTAGGGCGCGCCTTAACACTCTCCCCTGTTGAAGC -3' |
| Vκ1a: 5'-ATGGCAGCGGCTRACATCCAGATGACCCAG-3' |
| Vκ1b: 5'-ATGGCAGCGGCTGMCATCCAGTTGACCCAG -3' |
| Vκ1c: 5'- ATGGCAGCGGCTGCCATCCRGATGACCCAG -3' |
| Vκ1d: 5'- ATGGCAGCGGCTGTCATCTGGATGACCCAG -3' |
| Vκ2a: 5'- ATGGCAGCGGCTGATATTGTGATGACCCAG -3' |
| Vκ2b: 5'- ATGGCAGCGGCTGATRTTGTGATGACTCAG -3' |
| Vκ3a: 5'- ATGGCAGCGGCTGAAATTGTGTTGACRCAG -3' |
| Vκ3b: 5'- ATGGCAGCGGCTGAAATAGTGATGACGCAG -3' |
| Vκ3c: 5'- ATGGCAGCGGCTGAAATTGTAATGACACAG -3' |
| Vκ4a: 5'- ATGGCAGCGGCTGACATCGTGATGACCCAG -3' |
| Vκ5a: 5'- ATGGCAGCGGCTGAAACGACACTCACGCAG -3' |
| Vκ6a: 5'- ATGGCAGCGGCTGAAATTGTGCTGACTCAG -3' |
| Vκ6b: 5'- ATGGCAGCGGCTGATGTTGTGATGACACAG -3' |
| Vk/λ1: 5'- TTGTTATTGCTAGCTGCACAACCAGCAATGGCAGCGGCT -3' |
| Cλ1: 5'- AGGTAGGGCGCGCCTTATGAACATTCYGYAGGGGC -3' |
| Vλ1a: 5'- ATGGCAGCGGCTCAGTCTGTGCTGACTCAG -3' |
| Vλ1b: 5'- ATGGCAGCGGCTCAGTCTGTGYTGACGCAG -3' |
| Vλ1c: 5'- ATGGCAGCGGCTCAGTCTGTCGTGACGCAG -3' |
| Vλ2 : 5'- ATGGCAGCGGCTCAGTCTGCCCTGACTCAG -3' |
| Vλ3a: 5'- ATGGCAGCGGCTTCCTATGWGCTGACTCAG -3' |
| Vλ3b: 5'- ATGGCAGCGGCTTCCTATGAGCTGACACAG -3' |
| Vλ3c: 5'- ATGGCAGCGGCTTCTTCTGAGCTGACTCAG -3' |
| Vλ3d: 5'- ATGGCAGCGGCTTCCTATGAGCTGATGCAG -3' |
| Vλ4: 5'- ATGGCAGCGGCTCAGCYTGTGCTGACTCAA -3' |
| Vλ5: 5'- ATGGCAGCGGCTCAGSCTGTGCTGACTCAG -3' |
| Vλ6: 5'- ATGGCAGCGGCTAATTTTATGCTGACTCAG -3' |
| Vλ7: 5'- ATGGCAGCGGCTCAGRCTGTGGTGACTCAG -3' |
| Vλ8: 5'- ATGGCAGCGGCTCAGACTGTGGTGACCCAG -3' |
| Vλ4/9: 5'- ATGGCAGCGGCTCWGCCTGTGCTGACTCAG -3' |
| Vλ10: 5'- ATGGCAGCGGCTCAGGCAGGGCTGACTCAG -3' |

**TABLE III: Primers used for PCR amplification of the human variable regions of the heavy chain.**

| |
|---|
| VH1: 5'- ATTTACTCGAGTGAGGAGACGGTGACCAGGGTGCC -3' |
| VH2: 5'- ATTTACTCGAGTGAAGAGACGGTGACCATTGTCCC -3' |
| VH3: 5'- ATTTACTCGAGTGAGGAGACGGTGACCAGGGTTCC -3' |
| VH4: 5'- ATTTACTCGAGTGAGGAGACGGTGACCGTGGTCCC -3' |
| VH1A: 5'- TTACTCGCGGCCCAGCCGGCCATGGCCGCAGCT -3' |

**TABLE IV: Primers used for PCR amplification of the human variable regions of the light chains.**

| |
|---|
| Vκ1: 5'- TTATAGAGCTCGACATCCAGATGACCCAGTCTCC -3' |
| Vκ2: 5'- TTATAGAGCTCGATGTTGTGATGACTCAGTCTCC -3' |
| Vκ3: 5'- TTATAGAGCTCGAAATTGTGTTGACGCAGTCTCC -3' |
| Vκ4: 5'- TTATAGAGCTCGACATCGTGATGACCCAGTCTCC -3' |
| Vκ5: 5'- TTATAGAGCTCGAAACGACACTCACGCAGTCTCC -3' |
| Vκ6: 5'- TTATAGAGCTCGAAATTGTGCTGACTCAGTCTCC -3' |
| Vκ7: 5'- TATAAGCGGCCGCACGTTTGATTTCCACCTTGGTCCC -3' |
| Vκ8: 5'- TATAAGCGGCCGCACGTTTGATCTCCAGCTTGGTCCC -3' |
| Vκ9: 5'- TATAAGCGGCCGCACGTTTGATATCCACTTTGGTCCC -3' |
| Vκ10: 5'- TATAAGCGGCCGCACGTTTGATCTCCACCTTGGTCCC -3' |
| Vκ11: 5'- TATAAGCGGCCGCACGTTTAATCTCCAGTCGTGTCCC -3' |
| Vλ1: 5'- ATTTAGAGCTCCAGTCTGTGTTGACGCAGCCGCC -3' |
| Vλ2: 5'- ATTTAGAGCTCCAGTCTGCCCTGACTCAGCCTGC -3' |
| Vλ3: 5'- ATTTAGAGCTCTCCTATGTGCTGACTCAGCCACC -3' |
| Vλ4: 5'- ATTTAGAGCTCTCTTCTGAGCTGACTCAGGACCC -3' |
| Vλ5: 5'- ATTTAGAGCTCCACGTTATACTGACTCAACCGCC -3' |
| Vλ6: 5'- ATTTAGAGCTCCAGGCTGTGCTCACTCAGCCGTC -3' |
| Vλ7: 5'- ATTTAGAGCTCAATTTTATGCTGACTCAGCCCCA -3' |
| Vλ8: 5'- ATATTGCGGCCGCACCTAGGACGGTGACCTTGGTCCC -3' |
| Vλ9: 5'- ATATTGCGGCCGCACCTAGGACGGTCAGCTTGGTCCC -3' |
| Vλ10:5'-ATATTGCGGCCGCACCTAAAACGGTGAGCTGGGTCCC-3' |

**TABLE V. The amino acid sequence comparison of published IgE sequences reveals that the light chains of the known IgE antibodies binding to diverse groups of allergens are strikingly conserved. Conserved amino acids are shown in bold.**

| | | | | | | |
|---|---|---|---|---|---|---|
| allergen | Ig | clone | PDB | CDR-L1 | **CDR-L2** | **CDR-L3** |
| Bet v 1 (1) | IgE | C-H1 | | | | **QQ**SYSTP--RT |
| | | C-H2 | | | | AAWDDSLSG**R**VV |
| | | C-H3 | | | | **QQ**RSNW**P**-P**LT** |
| Phl p 1 (2) | IgE | 25 | | **SQ**S**I**GN------Y**L**N**WY** | **LLIYAASSLQS** | **QQ**SNRT**P**--I**TF** |
| | | 10 | | **SQ**TFNN------Y**L**N**WY** | LLIY**AA**STLRR | **QQ**SYST**P**--L**TF** |
| | | 43 | | **S**RTIYN------Y**L**N**WY** | LLIH**AA**ST**LQ**D | **QQ**SHGT**P**--L**TF** |
| Phl p 5 (3) | IgE | 31 | | **SQ**S**ISS**------Y**L**N**WY** | LLIY**AA**SSLQS | **QQ**SHST**P**--Y**TF** |
| | | 14 | | **S**H**SI**SN------Y**L**N**WY** | LLIY**AA**SSLQS | QESF**S**PS--G**TF** |
| | | 28 | | **SQ**S**I**LG------Y**L**N**WY** | LLIY**AAS**T**LQS** | **QQ**SYIT**P**--R**TF** |
| | | 5 | | **SQGISS**------W**L**A**WY** | LLIYS**ASSLQS** | **QQ**AN**S**F**P**--Y**TF** |
| hevein (4) | IgE | 1A4 | | **SQ**SV**SS**-----SY**LAWY** | LLIYG**ASS**RAT | **QQ**YG**S**S**P**--L**TF** |
| hevein (4) | IgE | 1C2 | | **SQ**SISS------Y**L**N**WY** | **LLIYAASSLQS** | **QQ**SY**S**T**P**--R**TF** |
| Bos d 5 (5) | IgE | D1 | | **SQGISS**------R**L**A**WY** | **LLIYAASSLQS** | **QQYHSYP**--**WTF** |

| | | | | | | |
|---|---|---|---|---|---|---|
| (1) Jakobsen et al. (2004) Isolation of high-affinity human IgE and IgG antibodies recognising Bet v 1 and Humicola lanuginose lipase from combinatorial phage libraries. Mol. Immunol. 41: 941-53. (2) Flicker et al.(2006) Spatial clustering of the IgE epitopes on the major timothy grass pollen allergen Phl p 1: Importance for allergenic activity. J. Allergy Clin. Immunol. 117: 1336-43. (3) Steinberger et al. (1996) Construction of a combinatorial IgE library from an allergic patient. Isolation and characterisation of human IgE Fabs with specificity for the major timothy grass pollen allergen, Phl p 5. J. Biol. Chem. 271: 10967-72. (4) Laukkanen et al. (2003) Hevein-specific recombinant IgE antibodies from human single-chain antibody phage display libraries. J. Immunol. Methods 278: 271-81. (5) Niemi *et al.* (2007) In press. | | | | | | |

### References

Argos, P. (1988) Protein Engineering 2, 101-113.
Borst, P., Zelcer, N. and van de Wetering, K. (2006) Cancer Letters, 234, 51-61.
Carter, P.J. (2006) Nature Reviews Immunology 6, 343-356.
De Genst E., Silence K., Decanniere K., Conrath K., Loris R., Kinne J., Muyldermans S., Wyns L. (2006) PNAS 103, 4586-4591.
Hoogenboom, H.R., de Bruïne, A.P., Hufton, S.E., Hoet, R.M., Arends, J.-W. and Roovers, R.C. (1998) Immunotechnolgy 4, 1-20.
Hoogenboom, H.R (2005) Nature Biotechnology 23, 1105-1116.
Laukkanen, M.-L., Mäkinen-Kiljunen, S., Isoherranen, K., Haahtela, T., Söderlund, H., and Takkinen, K. (2003 J. Immunol. Meth. 278, 271-281.
Mirza, O., Henriksen, A., Ipsen, H., Larsen, J.N., Wissenbach, M., Spangfort, M.D., and Gajhede, M. (2000) J. Immunol. 165, 331-338.
Prasad, L., Waygood, E.B., Lee, J.S. and Delbaere, L.T.J. (1998) J. Mol. Biol. 280, 829-845.

## Claims

1. Method of preparing a recombinant IgE monoclonal antibody or a Fab or scFv fragment thereof capable of preventing binding of a ligand to a drug-resistance pump, wherein the binding site of the ligand contains a depression, cleft, channel or a planar surface, and said site is weakly IgG or IgM immunoreactive; said method comprising the steps of
a) isolating total mRNA from IgE producing cells from a human derived sample;
b) synthesizing of the cDNAs encoding the IgE Fd gene region and kappa/lambda light chain genes based on the total mRNA obtained in step a) to create an IgE expression library;
c) screening the expressed library against a desired target protein or cell or particle expressing said target protein on its surface, wherein said target protein is said drug-resistance pump;
d) isolating clones from the library showing medium or high affinity over 10⁷M⁻¹ towards said protein;
e) selecting those clones obtained from step d) that bind to a depression, cleft, channel or a planar surface in the binding site of the ligand and prevent the binding of said ligand to said drug-resistance pump.

2. The method according to claim 1, wherein in step e) an IgE monoclonal antibody clone is selected, which clone prevents binding of all ligands to said drug-resistance pump.

3. The method according to claim 1, wherein said drug-resistance pump has more than one ligand.

4. The method according to claim 3, wherein in step e) an IgE monoclonal antibody clone is selected, which clone prevents binding of one specific ligand to said drug-resistance pump.

5. The method according to claim 1, wherein said drug-resistance pump is multidrug resistance associated protein 2, MRP2.

6. The method according to claim 5, wherein said ligand is conjugated or unconjugated organic anion, such as glutathione conjugates, glucuronide conjugates or leukotrines, or methotrexate, ochratoxin A or PAH.

7. The method according to claim 6, wherein said ligand is estradiol-17-b-D-glucuronide.

8. The method according to claim 5, wherein in step e) an IgE monoclonal antibody clone is selected, which clone prevents the binding of one or more ligands to MRP2 but do not prevent binding of ligands to ATP-binding cassette transporters, i.e. ABC-transporters.

9. The method according to claim 5, wherein in step e) an IgE monoclonal antibody clone is selected, which clone prevents the binding of one specific ligand to MRP2.

10. The method according to claim 1 further comprising the step of isolating the DNA encoding the IgE antibody obtained in step e).

## Patentansprüche

1. Verfahren zur Herstellung eines rekombinanten monoclonalen IgE-Antikörpers oder eines Fab- oder scFv-Fragments davon, das in der Lage ist, die Bindung eines Liganden an eine Drug-Resistenz-Pumpe (drug resistance pump) zu verhindern, wobei die Bindungsstelle des Liganden eine Vertiefung, eine Spalte, einen Kanal oder eine planare Oberfläche beinhaltet und die Stelle schwach IgG- oder IgM-immunreaktiv ist;
wobei das Verfahren die Schritte umfasst:
a) Isolieren von Gesamt-mRNA aus IgE-herstellenden Zellen aus einer Probe, die vom Menschen stammt;
b) Synthetisieren der cDNAs, die die IgE-Fd-Genregion und die kappa/lambda Gene der leichten Kette codieren, basierend auf der Gesamt-mRNA, die in Schritt a) erhalten wurde, um eine IgE-Expressionsbibliothek zu erstellen;
c) Screenen der exprimierten Bibliothek gegen ein gewünschtes Zielprotein oder eine Zelle oder ein Partikel, die/der das Zielprotein auf ihrer/seiner Oberfläche exprimiert, wobei das Zielprotein die Drug-Resistenz-Pumpe ist;
d) Isolieren von Klonen aus der Bibliothek, die mittlere oder hohe Affinität von mehr als 10⁷M⁻¹ gegenüber dem Protein zeigen;
e) Auswählen der in Schritt d) erhaltenen Klone, die an eine Vertiefung, eine Spalte, einen Kanal oder eine planare Oberfläche in der Bindungsstelle des Liganden binden und die die Bindung dieses Liganden an die Drug-Resistenz-Pumpe verhindern.

2. Verfahren nach Anspruch 1, wobei in Schritt e) ein monoclonaler IgE-Antikörper-Klon ausgewählt wird, dessen Klon die Bindung aller Liganden an die Drug-Resistenz-Pumpe verhindert.

3. Verfahren nach Anspruch 1, wobei die Drug-Resistenz-Pumpe mehr als einen Liganden hat.

4. Verfahren nach Anspruch 3, wobei in Schritt e) ein monoclonaler IgE-Antikörper-Klon ausgewählt wird, dessen Klon die Bindung eines spezifischen Liganden an die Drug-Resistenz-Pumpe verhindert.

5. Verfahren nach Anspruch 1, wobei die Drug-Resistenz-Pumpe das Multidrug-Resistenz-assoziierte Protein 2 (multidrug resistance associated protein 2 (MRP2)) ist.

6. Verfahren nach Anspruch 5, wobei der Ligand ein konjugiertes oder unkonjugiertes organisches Anion ist, wie z. B. Glutathion-Konjugate, Glucuronid-Konjugate oder Leukotriene, oder Methotrexate, Ochratoxin A oder PAH.

7. Verfahren nach Anspruch 6, wobei der Ligand Estradiol-17-b-D-Glucuronid ist.

8. Verfahren nach Anspruch 5, wobei in Schritt e) ein monoclonaler IgE-Antikörper-Klon ausgewählt wird, dessen Klon die Bindung von einem oder von mehreren Liganden an MRP2 verhindert, aber der nicht die Bindung von Liganden an ATP-bindende Kassetten-Transporter (ATP-binding cassette transporters), d. h. ABC-Transporter, verhindert.

9. Verfahren nach Anspruch 5, wobei in Schritt e) ein monoclonaler IgE-Antikörper-Klon ausgewählt wird, dessen Klon die Bindung eines spezifischen Liganden an MRP2 verhindert.

10. Verfahren nach Anspruch 1, des Weiteren umfassend den Schritt der Isolierung der DNA, die den IgE-Antikörper codiert, der in Schritt e) erhalten wurde.

## Revendications

1. Procédé de préparation d'un anticorps monoclonal IgE recombinant ou d'un fragment Fab ou scFv de celui-ci capable d'empêcher la liaison d'un ligand à une pompe de résistance aux médicaments, dans lequel le site de liaison du ligand contient une dépression, une crevasse, un canal ou une surface plane, et ledit site est faiblement immunoréactif à une IgG ou à une IgM ; ledit procédé comprenant les étapes suivantes :
a) l'isolement de l'ARNm total provenant de cellules productrices d'IgE issues d'un échantillon dérivé d'un humain ;
b) la synthèse des ADNc codant la région du gène Fd de l'IgE et les gènes des chaînes légères kappa/lambda sur la base de l'ARNm total obtenu dans l'étape a) pour créer une banque d'expression d'IgE ;
c) le criblage de la banque d'expression contre une protéine cible souhaitée ou une cellule ou particule exprimant ladite protéine cible sur sa surface, dans lequel ladite protéine cible est ladite pompe de résistance aux médicaments ;
d) l'isolement de clones provenant de la banque présentant une affinité moyenne à élevée supérieure à 10⁷M⁻¹ envers ladite protéine ;
e) la sélection des clones obtenus dans l'étape d) qui se lient à une dépression, une crevasse, un canal ou une surface plane dans le site de liaison du ligand et qui empêche la liaison dudit ligand à ladite pompe de résistance aux médicaments.

2. Procédé selon la revendication 1, dans lequel, dans l'étape e), un clone d'anticorps monoclonal IgE est sélectionné, ledit clone empêchant la liaison de tous les ligands à ladite pompe de résistance aux médicaments.

3. Procédé selon la revendication 1, dans lequel ladite pompe de résistance aux médicaments a plus d'un ligand.

4. Procédé selon la revendication 3, dans lequel, dans l'étape e), un clone d'anticorps monoclonal IgE est sélectionné, ledit clone empêchant la liaison d'un ligand spécifique à ladite pompe de résistance aux médicaments.

5. Procédé selon la revendication 1, dans lequel ladite pompe de résistance aux médicaments est la protéine 2 associée à la résistance pléïotrope MRP2.

6. Procédé selon la revendication 5, dans lequel ledit ligand est un anion organique conjugué ou non conjugué, tel que des conjugués de glutathione, des conjugués de glucuronide ou des leucotrines, ou le méthotréxate, l'ochratoxine A ou le PAH.

7. Procédé selon la revendication 6, dans lequel ledit ligand est l'estradiol-17-b-D-glucuronide.

8. Procédé selon la revendication 5, dans lequel, dans l'étape e), un clone d'anticorps monoclonal IgE est sélectionné, ledit clone empêchant la liaison d'un ou de plusieurs ligands à MRP2, mais n'empêchant pas la liaison des ligands aux transporteurs à boîtes de liaison à l'ATP, c'est-à-dire les transporteurs ABC.

9. Procédé selon la revendication 5, dans lequel, dans l'étape e), un clone d'anticorps monoclonal IgE est sélectionné, ledit clone empêchant la liaison d'un ligand spécifique à MRP2.

10. Procédé selon la revendication 1, comprenant en outre l'étape d'isolement de l'ADN codant l'anticorps IgE obtenu dans l'étape e).
